# EUROPEAN PATENT SPECIFICATION

(11) **EP 0 341 007 B1**
(45) Date of publication and mention of the grant of the patent: **14.09.1994**
(21) Application number: 89304371.1
(22) Date of filing: 28.04.1989
(51) Int. Cl.: A61L 25/00

(54) **Surgical adhesive material**
Chirurgisches Klebstoffmaterial
Matériau pour adhésif chirurgical

(30) Priority: 02.05.1988 US 189187
(43) Date of publication of application: 08.11.1989
(73) Proprietor: PROJECT HEAR, Palo Alto California 94304 (US); MATRIX PHARMACEUTICAL INC., Menlo Park California 94025 (US)
(72) Inventor: Sierra, David H., Birmingham Alabama 35222 (US); Luck, Edward E., Menlo Park California 94025 (US); Brown, Dennis M., Menlo Park California 94025 (US)
(74) Representative: Harrison, David Christopher

(56) References cited:
- EP-A- 0 068 047
- EP-A- 0 068 048
- EP-A- 0 068 149
- EP-A- 0 147 021
- EP-A- 0 166 263
- WO-A-86/01814
- GB-A- 2 041 942
- US-A- 4 600 574

## Description

The field of the present invention relates to surgical adhesives.

Early surgical adhesive formulations based on fibrinogen suffered from a number of disadvantages. The fibrin solutions necessarily contained a high content of fibrinogen, about 8-10%, which could only be prepared from fibrinogen lyophilizates with difficulty. This so-called cryoprecipitate was relatively unstable and had to be stored at temperatures below -20°C until used. Formulations to improve the stability of the cryoprecipitate included adding inhibitors of plasminogen activator or albumin. The fibrinogen concentrates were mixed with thrombin just prior to application to the wound.

Other formulations of surgical adhesives had a pore structure based on collagen for covering wounds wherein a non-woven fabric consisting of collagen fibers was applied to the wound. The fabric was fixed to the wound using a fibrinogen-thrombin mixture which was applied to either the inner or outer side of the collagen fabric. However, the fibrinogen was found to coagulate rapidly and thus not to effectively penetrate into the collagen fabric.

The current formulations use patient autogenous fibrinogen glues together with thrombin. While the use of autogenous fibrinogen avoids problems with rejection of the material, the adhesives require relatively large quantities of patient blood. Further, the processing times range from an hour to overnight, and require both the equipment and expertise of a hospital clinical blood laboratory with trained technicians. Additionally, a number of reagents are introduced into the blood to fractionate and concentrate the fibrinogen and related proteins from the plasma in some patient autogenous fibrin glue (AFG) formulations.

### Relevant Literature

U.S. Patent No. 4,650,678 describes a solid fibrinogen formulation which contains a substance having a urea or guanidine radical to increase the solubility and viscosity of fibrinogen solutions. U.S. Patent No. 4,600,574 describes a surgical adhesive based on a flat material consisting of collagen, gelatin or polysaccharide impregnated with a solution of fibrinogen and Factor XIII, which material is lyophilized to form a matrix.

### SUMMARY OF THE INVENTION

The present invention provides a surgical adhesive comprising, in an aqueous composition, patient autogenous plasma, collagen, thrombin, and optionally, an antifibrinolytic agent. The present adhesive is formed from the patient's plasma without the use of any added reagents for concentration or isolation of the fibrinogen. Conveniently, the adhesive is formulated as a two-part composition which is mixed together just prior to use.

### DESCRIPTION OF SPECIFIC EMBODIMENTS

A surgical adhesive using patient autogenous plasma in conjuction with collagen and thrombin is provided. The plasma can be used directly or as a plasma cryoprecipitate in which the fibrinogen has been concentrated without the use of any added reagents.

The surgical adhesive comprises in an aqueous composition patient autogenous plasma, collagen in an amount sufficient to thicken the composition, thrombin in an amount sufficient to catalyze polymerization of fibrinogen present in the plasma and, optionally, an antifibrinolytic agent in an amount sufficient to retard degradation of the resulting adhesive clot. The surgical adhesive is conveniently formulated as a two-part composition in which plasma and collagen constitute the first component, and thrombin together with an antifibrinolytic agent, constitutes the second component.

Patient autogenous plasma provides a source of fibrinogen which provides the adhesive component of the composition. The plasma can be used "as it is" after standard preparation which includes centrifuging out cellular components of blood. Alternatively, the plasma can be further processed to concentrate the fibrinogen to prepare a plasma cryoprecipitate. The plasma cryoprecipitate can be prepared by freezing the plasma for at least about an hour at about -20°C, and then storing the frozen plasma overnight at about 4°C to slowly thaw. The thawed plasma is centrifuged and the plasma cryoprecipitate is harvested by removing approximately four-fifths of the plasma to provide a cryoprecipitate comprising the remaining one-fifth of the plasma. Optionally, the plasma cryoprecipitate can be mixed with an equal volume of approximately 10% CaCl₂ solution. Approximately 0.5 ml to 1.0 ml of either the plasma or the plasma-cryoprecipitate provides 1 to 2 ml of adhesive composition which is sufficient for use in middle ear surgery.

Collagen, preferably hypoallergenic collagen, is present in the adhesive in an amount sufficient to thicken the adhesive composition. The collagen may be atelopeptide collagen or not. In addition to thickening the composition, the collagen augments the fibrin by acting as a macromolecular lattice work or scaffold to which the fibrin network adsorbs. This gives more strength and durability to the resulting glue clot with a relatively low concentration of fibrinogen in comparison to the various concentrated autogenous fibrinogen glue formulations (i.e. AFGs). The amount of the collagen can be varied to provide adhesives of differing viscosities and strengths, depending on the particular application for the adhesive. Usually, the collagen is a flowable composition dispersed in phosphate buffered saline to provide a final concentration in the adhesive formulation of from 5 mg/ml to 30 mg/ml, more usually 10 mg/ml to 20 mg/ml, most usually 15 mg/ml. Collagen is commercially available from a variety of sources, including Collagen Corporation (which sells hypoallergenic collagen under the tradename Zyderm). Collagen is generally available in a soft paste form, packaged in a sterile syringe. Further descriptions of collagen are found in U.S. Patent No. 4,233,360.

Thrombin acts as a catalyst for fibrinogen to provide fibrin, an insoluble polymer. Thrombin is present in the surgical adhesive in an amount sufficient to catalyze polymerization of fibrinogen present in the patient plasma. Usually the thrombin is present in the adhesive composition in a concentration of from 1 to 1000 NIH units (NIHu) of activity, usually 100 to 500 NIHu, most usually 200 to 300 NIHu. The thrombin can be from a variety of host animal sources, conveniently bovine. Thrombin is commercially available from a variety of sources including Parke Davis, usually lyophilized with buffer salts and stabilizers in vials which provide thrombin activity ranging from 1000 NIHu to 10,000 NIHu. The thrombin is usually prepared by reconstituting the powder by the addition of either sterile distilled water or isotonic saline.

Usually the surgical adhesive will additionally comprise an effective amount an antifibrinolytic agent to enhance the integrity of the glue clot as the healing processes occur. A number of antifibrinolytic agents are well known and include aprotinin, Cl-esterase inhibitor and ε-aminocaproic acid (EACA). ε-aminocaproic acid, the only antifibrinolytic agent approved by the FDA, is effective at a concentration of from 5 mg/ml to 40 mg/ml of the final adhesive composition, more usually from 20 to 30 mg/ml. EACA is commercially available as a solution having a concentration of 250 mg/ml. Conveniently, the commercial solution is diluted with distilled water to provide a solution of the desired concentration. That solution is desirably used to reconstitute lyopholized thrombin to the desired thrombin concentration.

For dental or orthopedic applications, inorganic minerals or mixture of inorganic minerals, naturally occuring or synthetic, desirably hydroxyapatite or minerals found in bone powder or chips may be added to the formulation, most conveniently to the plasma fraction of component 1. The mineral(s) are present in a volume ratio to the collagen component of from 1:1 to 4:1 depending upon the desired flow characteristics or intended use and site. Additionally, viable osteoblasts may be harvested from a donor site and incorporated into the composition, conveniently in component 1, for use in transplantation.

The surgical adhesive may additionally contain an antibiotic. The antibiotic may be incorporated into the collagen component if the antibiotic is a liquid. Alternatively, the antibiotic may be suspended in the plasma fraction of component 2 if it is in powder form. The therapeutic dose levels of a wide variety of antibiotics for use in drug release systems are well known. See for example, Collagen, Vol. III, Biotechnology; Marcel E. Nimni, Ph.D., Editor, CRC Press, Inc. (1988) pp. 209-221, and the references cited therein. Anti-microbial agents are particularly useful for compositions applied to exposed wound repair sites such as sites in the mouth or to compromised wound sites such as burns.

The surgical adhesive is conveniently formed by mixing two components just prior to use. The first component comprises the autogenous plasma together with collagen. That component is conveniently prepared by mixing the plasma with collagen to form a substantially uniform composition under low or no shear conditions at ambient or lower temperatures. Conveniently, using two syringes joined by a syringe-to-syringe connector having about a 1 mm or less diameter opening, substantial uniformity can be achieved with simple, generally available equipment. Generally, about 5 to 10 passes through the opening is sufficient. This component can be prepared during surgery or up to 8 hrs. prior to surgery, when stored at room temperature. Alternately, the plasma fraction may be collected and prepared up to one week prior to mixing with the collagen fraction. The second component comprises thrombin. If an antifibrinolytic agent is present in the composition, it is usually mixed with the thrombin as part of component 2. Component 2 can be stored for about 8 hrs. at room temperature, for about 2 days at about 4°C. or for up to a week when frozen at -20°C.

The two components are mixed just prior to the application to the patient. The components may be formulated with concentrations that allow mixing the components in substantially equal volumes to simplify the final preparation of the adhesive. Conveniently, a dual-syringe holder with a disposable mixing tip can be used. Alternatively, the two components can be mixed using two syringes as described above.

The surgical adhesive can be used in applications where prior art surgical adhesives were previously used. The material can be used as a soft tissue augmentor or soft tissue substitute in plastic reconstructive surgery. The adhesive may be also used to attach skin grafts to a recipient site without the use of sutures or with a reduced number of sutures, or as a growth matrix for transplanted intact osteoblasts in bone repair and reconstruction. The adhesive can also be used for applications such as ossicular chain reconstruction, nerve anastomosis or other situations where repair by sutures is impossible or undesirable, or as a wound dressing. The surgical adhesive may be applied in a number of ways determined by the surgical indication and technique.

The following examples are offered by way of illustration and not by way of limitation.

### EXPERIMENTAL

### Example 1

### Preparation of Adhesive

To prepare a preferred formulation of the adhesive composition, the following protocol was followed. Patient's blood (5 cm³) was collected in a citrated vacuum blood collection tube (Vacutainer) by venipuncture. The blood was centrifuged for 10 min at 4000 rpm. About 0.5 cm³ plasma was removed from the Vacutainer with a 1 cm³ syringe. A syringe-to-syringe connector (20-gauge) was used to mix 0.5 cm³ collagen (Zyderm I, Collagen Corporation) with the 0.5 cm³ patient's plasma for about 5 to 10 passes. Component 1 was then ready for use or was stored for up to about 6 to 8 hrs. at room temperature.

Component 2 was prepared by drawing 1 cm³ of a solution of 250 mg/ml of aminocaproic acid into a 12-cm³ syringe. Nine cm³ of Water for Injection, U.S.P., was drawn into the same syringe to provide a 25 mg/ml concentration of aminocaproic acid. Two cm³ of that solution was added to 1000 NIH units of bovine thrombin (Thrombostat, Parke-Davis) and drawn into a 1 cm³ syringe. Component 2 was ready for use or was stored, as described previously.

At that point, either the two syringes were mounted into a dual-syringe dispensor/mixer or modified spinal tap needles were attached to the individual syringes to mix and apply the components.

### Example 2

### Concentrations and Gelation Time Effects In Vitro

The following materials were used to determine gelation time in vitro:
- lyopholized fibrinogen from Sigma Chemical Company, bovine source, cat. #F4753, reconstituted to desired concentrations with Ringer's solution;
- collagen Zyderm Collagen Implant 1 without lidocaine by Collagen Corporation at 35 mg/ml, diluted as needed by Ringer's solution; and
- lyopholized thrombin Thrombostat by Parke-Davis, reconstituted with Sterile Water for Injection, U.S.P. to 100 NIH units per ml. (1 NIH unit (10 µl) was delivered to each test formulation.)
Human plasma was collected using citrated Vacutainers, then centrifuged for 10 min at 4000 rpm. A 1 ml test solution of each formulation was maintained at 37°C using a circulating water bath in each assay.

Tables 1 and 2 illustrate the results. As used in the Tables, [Fibro] [collagen] indicate the concentration of clottable lyopholized fibrinogen and collagen, respectively, dissolved in Ringer's Solution and Gel time is the amount of time for gelation of the clot. The collagen and fibrinogen concentrations listed are the final concentrations in the test adhesive mixture.

**TABLE 1**

| LYOPHOLIZED FIBRINOGEN WITHOUT COLLAGEN | | |
|---|---|---|
| [Fibro] (mg/ml) | Gel Time (sec) | Comments |
| 5 | 19 | solid clot |
| 2.5 | 22 | solid clot |
| 1.25 | 30 | solid clot |
| 0.6 | 40 | solid clot |
| 0.3 | 50 | solid clot |
| 0.15 | 90 | fluid clot, transparent |
| 0.07 | 210 | fluid clot, fragile |
| 0.035 | 270 | fluid clot, fragile |
| 0.017 | -- | no visible clot formation |

**TABLE 2**

| LYOPHOLIZED FIBRINOGEN WITH COLLAGEN AT 6 mg/ml | | |
|---|---|---|
| [Fibro] (mg/ml) | Gel Time (sec) | Comments |
| 2.5 | 7 | solid clot |
| 1.25 | 8 | solid clot |
| 0.6 | 9 | solid clot |
| 0.3 | 14 | solid clot |
| 0.15 | 20 | solid clot |
| 0.07 | -- | no apparent clotting effect |
| 0.035 | -- | no apparent clotting effect |

As demonstrated using fibrinogen without collagen (Table 1), there was some clotting effect at 0.07 and 0.035 mg/ml fibrinogen concentration. However, due to the fragility and fluidity of the resultant clots and comparison to the viscosity of a collagen-alone control using 6 mg/ml collagen, it was difficult to discern any clotting effect at these lower fibrinogen concentrations. As shown in Table 2, solid clots were achieved at fibrinogen concentrations of from 2.5 to 0.15 mg/ml using 6 mg/ml collagen.

**TABLE 3**

| LYOPHOLIZED FIBRINOGEN WITH COLLAGEN AT 15 mg/ml | | |
|---|---|---|
| [Fibro] (mg/ml) | Gel Time (sec) | Comments |
| 2.5 | -- | instantaneous gelation |
| 1.25 | 10 | solid clot |
| 0.6 | 15 | solid clot |
| 0.3 | 25 | solid clot |
| 0.15 | 30 | solid clot |
| 0.07 | 40 | fluid and fragile clot |
| 0.035 | 45 | fluid and fragile clot |
| 0.017 | 120 | fluid and fragile clot |
| 0.008 | -- | no apparent gelation |

This data demonstrated the practical limits for concentrations that can be used under these experimental conditions for reconstituted lyopholized bovine fibrinogen. In particular, compositions with concentrations of lyopholized fibrinogen as high as 2.5 mg/ml gelled instantly and were not useful. Concentrations of 0.07 mg/ml or less produced a fluid and fragile clot. Formulations using between 1.25 and 0.15 mg/ml fibrinogen together with 15 mg/ml collagen produced useful adhesive compositions. Higher concentrations than 15 mg/ml of collagen may be used successfully. However, it is difficult to objectively assess gelation time due to the limitations of current test equipment in evaluating test solutions of relatively high viscosity.

A study was performed to determine the dilutions of plasma that were sufficient for proper clot formation. The results are shown in Table 4. Other than the plasma dilution, the conditions were the same as those described for the studies illustrated in Tables 1-3.

**TABLE 4**

| PLASMA WITH RINGER'S AS DILUENT | | | |
|---|---|---|---|
| Plasma ml | Ringer's ml | Gel Time sec | Comments |
| 1.0 | 0 | 15 | solid clot |
| 0.75 | 0.25 | 12 | solid clot |
| 0.5 | 0.5 | 20 | solid clot |
| 0.33 | 0.67 | 30 | fluid clot |
| 0.25 | 0.75 | 35 | fluid clot |
| 0.12 | 0.87 | -- | no gelation, small clots |

The purpose of this study was to compare the effect of various plasma dilutions. Since one is, in essence, diluting the plasma with collagen to form the adhesive, the effect of collagen in comparison to buffer solution as diluents on gelation time and clot quality was studied. Plasma was mixed with ZCI collagen (35 mg/ml) to determine useful ranges of plasma fibrinogen concentrations.

**TABLE 5**

| PLASMA WITH ZCI COLLAGEN AT A CONCENTRATION OF 35 mg/ml | | | | |
|---|---|---|---|---|
| Plasma (ml) | Collagen (ml) | [Collagen] (mg/ml) | Gel Time (sec) | Comments |
| 0.75 | 0.25 | 8.75 | 15 | * |
| 0.5 | 0.5 | 17.5 | 10 | solid clot |
| 0.33 | 0.67 | 23.1 | 30 | solid clot |
| 0.25 | 0.75 | 26.2 | 100 | small clots |

| | | | | |
|---|---|---|---|---|
| *Collagen congealed into a small clot within the gel, which was difficult to evaluate. | | | | |

As shown in Table 5, combining volumes of plasma with 35 mg/ml collagen in ratios between 1:1 to 1:2 plasma:collagen produced useful adhesives. This study demonstrated that the presence of collagen as a plasma "diluent" actually allows plasma fibrinogen to form a solid clot at lower fibrinogen concentrations than plasma fibrinogen at the same concentration but without collagen.

The effect of the calcium ion concentration on both the gelation time and clot quality was examined. Lyopholized bovine fibrinogen (3 mg/ml) was solubilized in various CaCl₂ solutions. No collagen was used in this study. Standard conditions were used (i.e. 37°C test temperature, 1 ml test solution used per [Ca⁺⁺] dose level, 1 NIHu thrombin).

**TABLE 6**

| EFFECT OF Ca⁺⁺ ON GELATION TIME, | | |
|---|---|---|
| [Ca⁺⁺] (M) | Gel Time (sec) | Comments |
| 0.01 | 30 | fast onset, slow to harden, fragile |
| 0.02 | 15 | firm clot |
| 0.04 | 10 | firm clot |
| 0.06 | 50 | soft clot at 25 sec, then firm |
| 0.08 | 150 | small clots on side of tube, then entire test solution gels |
| 0.10 | 150 | small clots on side of tube, then entire test solution gels |
| 0.20 | 10 min | |
| 0.40 | >10 min | |
| 0.80 | >10 min | |
| Ringer's control | 21 sec | [Ca⁺⁺] = 0.02 M |

The results illustrated in Table 6 show that the calcium ion concentration for optimum gelation time and clot quality ranges between 0.02 M and 0.04 M. This is in agreement with prior published studies.

### Example 3

### Shelf Life Effects on Gelation

The reagents and gelation conditions used in the study were identical to those described in Example 2.

Lyopholized fibrinogen was reconstituted in Ringer's in the following concentrations:

### Concentrations of Test Solutions

- fibrinogen: 3 mg/ml in test solution;
- fibrinogen with collagen:
   collagen: 17.5 mg/ml in test solution;
   fibrinogen: 1.5 mg/ml in test solution.

### Standard Gelation Conditions Used

test temperature: 37°C
amount of test solution: 1 ml
amount of thrombin: 1 NIHu
(10 µl of a 100 NIHu solution in sterile water for injection)

**TABLE 7**

| BOVINE FIBRINOGEN--GELATION TIME | | |
|---|---|---|
| Time Temp Exp (hrs) | 4°C Storage Temp | |
| | -ZCIl (sec) | +ZCIl (sec) |
| 0 | 20 | 10 |
| 24 | 19 | 10 |
| 48 | 18 | 8 |
| 72 | 20 | 10 |
| 96 | 15 | 10 |
| 120 | 15 | 10 |
| 168 | 15 | 10* |

| | | |
|---|---|---|
| *The fibrinogen appears gelled, even when thawed to 37°C prior to addition of thrombin. | | |
| Time Temp Exp (hrs) = time of test solution's exposure (or storage) to indicated temperatures (either 22°C or 4°C) -ZCIl = without ZCIl, [bovine fibrinogen solution without collagen] +ZCIl = with ZCIl [bovine fibrinogen solution mixed with collagen] | | |

**TABLE 8**

| HUMAN PLASMA--GELATION TIME | | | | | |
|---|---|---|---|---|---|
| Time Temp exp (hrs) | 4°C Storage Temp | | Time Temp exp (hrs) | 22°C Storage Temp | |
| | -ZCIl (sec) | +ZCIl (sec) | | -ZCIl (sec) | +ZCIl (sec) |
| 0 | 18 | 12 | 0 | 17 | 10 |
| 1 | 15 | * | 1 | 18 | 11 |
| 3 | 16 | * | 2 | 17 | 11 |
| 4 | 15 | * | 4 | 14 | 10 |
| 20 | 20 | * | 8 | 17 | 12 |
| 30 | 15 | * | 24 | 20 | * |
| 48 | 18 | * | | | |
| 120 | 17 | * | | | |

| | | | | | |
|---|---|---|---|---|---|
| *Test solution appeared to have gelled prior to addition of thrombin. | | | | | |

As demonstrated in the tables, lyopholized fibrinogen with collagen was stable for over 120 hrs at 4°C. Human plasma (normal range of fibrinogen concentration in plasma is about 2 to 4 mg/ml) with collagen gelled spontaneously in less than an hour at 4°C. At room temperature (22°C.), the mixture was stable for up to about 8 hrs.

### Example 4

### Clot Stability In Vitro

One ml of adhesive material was placed in about 30 ml of Ringer's solution and allowed to sit undisturbed at 37°C until the clot dissolved in its entirety. The adhesive materials compared were:
#1 - 0.5 ml human plasma + 0.5 ml ZCIl collagen + 100 NIH units bovine thrombin in 1 ml of 25 mg/ml ε-aminocaproic acid;
#2 - 0.5 ml human plasma + 0.5 ml ZCIl collagen + 100 NIH units bovine thrombin in 1 ml of Sterile Water for Injection, U.S.P.;
#3 - 1.0 ml human plasma + 100 NIH units bovine thrombin in 1 ml of 25 mg/ml ε-aminocaproic acid; and
#4 - 1.0 ml human plasma + 100 NIH units bovine thrombin in 1 ml Sterile Water for Injection, U.S.P.
Six replicate clots were formed for each of the four groups. One Ringer's solution control was used for a turbidity assessment.

The data shows that after 35 days, group #1 showed the least degradation, where 2 of 6 clots gave off very small amounts of flocculant material. Group #2 was the second most durable group of clots with 4 of 6 clots which gave off flocculant material. Group #3 was less stable than group #2, with 6 out of 6 clots giving off flocculant material. Group #4 showed the most degradation, with 2 out of 6 clots completely degraded and the remaining clots far more fluid and flocculant than the other groups.

### Example 5

### Human Clinical Trials

Human clinical trials included 24 patients undergoing a variety of middle ear surgical procedures, as well as one neurologic procedure. The 24 cases were broken down into two phases. Phase I incorporated the use of patient autologous plasma cryoprecipitate with collagen, thrombin and an anti-fibrinolytic agent. Twenty-one subjects were assigned to this first phase. Phase II incorporated the use of patient autologous citrated plasma (without any fibrinogen concentration) with collagen, thrombin and antifibrinolytic. Three subjects have been incorported into this phase. The surgical procedures and outcome for each test subject in both phases are outlined hereinafter. The preparative steps for each phase is as follows. For Phase I, 10 cm³ of the patient's blood was collected into two citrated 5 cm³ Vacutainers. The blood was centrifuged for 10 min at 4000 rpm. Plasma was collected from each tube and transferred to one siliconized Vacutainer using a 20-gauge x 3" spinal needle and a 12 cm³ syringe. Plasma was stored for 1 hr or more at -20°C, either in a cryofreezer or a circulating acetone bath. Frozen plasma was stored overnight at 4°C to slowly thaw.

On the morning of surgery, thawed plasma with precipitate was centrifuged for 10 min at 4000 rpm. The top about 4.5 ml of the clear plasma fraction was removed with a 20-gauge x 3" spinal needle and 12 cm³ syringe, leaving the bottom 0.5 ml of plasma with the cryoprecipitate in the Vacutainer tube. Then, 0.5 ml of 10% CaCl₂ solution was added to the cryoprecipitate-containing plasma and mixed thoroughly using a vortex mixer. After mixing, 0.5 ml of the cryoprecipitate/CaCl₂ solution was removed using a 1 cm³ tuberculin syringe. That syringe was connected to another 1 cm³ syringe containing 0.5 ml of ZCIl collagen without lidocaine (35 mg/ml) using a syringe-to-syringe connector, and mixed for 5 to 7 passes until the mixture was uniform. The preparation of component 1 was complete. Component 2 was produced as described in Example 1.

For Phase II, about 5 cm³ of the patient's blood was collected in one citrated 5 cm³ Vacutainer during the surgical procedure. Usually, the blood was collected by the anesthesiologist from a catheter access port. The collected blood in the citrated Vacutainer was then centrifuged in a clinical centrifuge for 10 minutes at 4000 rpm. 0.5 cm³ of the plasma fraction was then withdrawn with a 20g X 3" spinal needle and a 1 cm³ tuberculin syringe. The spinal needle was removed and a sterile syringe-to-syringe connector was attached. The syringe containing 1 cm³ of ZCIl (without lidocaine) was then attached to the other end of the connector. 0.5 cm³ of the collagen were then passed to the syringe containing the 0.5 cm³ of citrated plasma. The collagen-containing syringe was removed from the connector and an empty 1 cm³ syringe was attached. The plasma/collagen mixture was mixed by passing back and forth for 5 to 10 passes until uniform. Component 2 was produced as described in Example 1. The two component-bearing syringes were then attached to a dual-syringe deliver device, or modified spinal needles were attached to each individual syringe.

**TABLE 9**

| OUTLINE OF SURGERIES, PHASE I | | | | |
|---|---|---|---|---|
| Patient Case # | Surgical Procedure | Patient Age (at time of surgery) | Patient Sex | No. of Post-Op Exams (to date) |
| 1 | tympanossiculoplasty | 56 | F | 3 |
| 2 | ossiculoplasty revision | 62 | M | 2 |
| 3 | tympanoplasty with mastoidectomy | 9 | M | 3 |
| 4 | ossiculoplasty revision with meatoplasty revision | 24 | F | 3 |
| 5 | laser stapedotomy | 42 | M | 0 |
| 6 | tympanossiculoplasty | | M | |
| 7 | laser stapedotomy | | M | |
| 8 | ossiculoplasty | 14 | F | 2 |
| 9 | laser stapedotomy | 17 | M | |
| 10 | external canal exotoses | 39 | M | 3 |
| 11 | laser stapedotomy | 58 | M | 2 |
| 12 | laser stapedotomy | 43 | F | 2 |
| 13 | laser stapedotomy | 47 | F | 2 |
| 14 | ossiculoplasty | 10 | F | 2 |
| 15 | tympanomastoid reconstruction | 65 | F | 1 |
| 16 | ossiculoplasty | 68 | M | 1 |
| 17 | ossiculoplasty | | M | |
| 18 | laser stapedotomy | | F | |
| 19 | laser stapedotomy | 56 | F | 2 |
| 20 | laser stapedotomy | 49 | M | 1 |
| 21 | tympanossiculoplasty with mastoidectomy | | M | |

**TABLE 10**

| OUTLINE OF SURGERIES, PHASE II | | | | |
|---|---|---|---|---|
| Patient Case # | Surgical Procedure | Patient Age (at time of surgery) | Patient Sex | No. of Post-Op Exams (to date) |
| 22 | laser stapedotomy | 44 | F | 2 |
| 23 | ossiculoplasty | 29 | M | 2 |
| 24 | acoustic neuroma | 40 | F | 1 |

### Surgical Procedure Descriptions

External Canal Exotoses: The posterior canal wall skin was elevated and the bony exotoses were removed by use of a pneumatic hand-held de-burring tool. The canal wall skin was then placed back down using the surgical adhesive to adhere it to the bony wall as well as to fill in any gaps in the canal wall skin flaps.

Laser Stapedotomy: The tympanic membrane was dissected partially out of the sulcus, along with some of the adjacent canal wall skin. The cruces of the stapes was removed from the footplate by laser dissection. A small hole was then drilled into the center of the stapes footplate by laser. The teflon end of a stapes prosthesis was then introduced into the hole and was glued into place with the surgical adhesive. The platinum wire end of the prosthesis was then wrapped around the incus. Then, the tympanic membrane flap was re-positioned into the sulcus and was adhered in place with the surgical adhesive. No canal packing was used to hold the tympanic membrane in place.

Ossiculoplasty (revision): Generally, an incision was made immediately behind the ear. The tympanic membrane, along with a flap of adjacent canal wall skin was incised and folded up to allow for good exposure and access to the middle ear space. Depending upon the condition of the ossicles, prosthesis replacing either one, two or all three ossicles were attached to either the tympanic membrane and/or ossicles and glued into position with the surgical adhesive. The tympanic membrane and attached canal wall skin flap were re-positioned and adhered back to their original configuration. The post-auricular incision was then closed with sutures. No canal packing was used to hold the tympanic membrane in place. Revision indicates that this was a repeat of a particular procedure for a patient.

Ossiculoplasty (revision) with Meatoplasty Revision: The same procedures were followed as described previously, however, prior to replacing the ossicle(s), a number of small incisions were made in the cartilagenous opening of the auditory canal (the mectus) as well as along the canal wall. Pieces of cartilage and small pieces of skin were excised as desired to re-shape and re-contour a deformed meatus and canal. The incisions were re-positioned and adhered with the surgical adhesive. Exposed gaps in the skin closures were filled with the adhesive to close the wounds. The ossiculoplasty then continued as previously described. After the ossiculoplasty procedure, the meatoplasty was again checked to ensure that the desired meatus and canal configuration were maintained and were subsequently packed with antibiotic-soaked dressing.

Tympanossiculoplasty: This is another version of the previously described ossiculoplasty. However, the tympanic membrane was replaced with either a homograph tympanic membrane transplant or a piece of patient autologous shaped, dried and sometimes formaldehyde-fixed fascia from the temporalis muscle. The ossicular prosthesis was positioned and glued onto the replacement tympanic membrane. Also, the replacement membrane was glued into position in the sulcus with the surgical adhesive. No canal packing was used.

Tympanossiculoplasty with Mastoidectomy: In addition to performing the above-described tympanossiculoplasty, part of the mastoid bone immediately posterior to the canal wall and middle ear space was rebuilt. Infected mastoid bone was debrided by a pneumatic hand-held drill after exposure of the site by an incision behind the ear. The subsequently formed cavity was then walled off from the middle ear space as well as simultaneously rebuilding the posterior canal wall by a piece of pre-formed, formaldehyde-fixed dura. Osteoblasts were harvested from a donor site in the mastoid bone and were mixed with the surgical adhesive either in situ or during the preparation of the adhesive. This adhesive/osteoblast mixture was then used to hold the pre-formed dura in place. The canal wall skin was then re-positioned and adhered to the dura wall with surgical adhesive after gluing the ossicular prosthesis and homograft tympanic membrane in place.

Tympanomastoid Reconstruction: This procedure was the same as described previously in the tympanossiculoplasty with mastoidectomy except that was no replacement of the ossicles.

Acoustic Neuroma: An approximately 5 cm diameter hole was drilled into the skull behind the ear. The dura immediately beneath the opening was incised and folded away to reveal the cerebellum and brain stem. The tumorous growth on the acoustic nerve (8th cranial nerve) was located and dissected away with a laser and microdissection instruments. A piece of patient autologous muscle and fascia were coated with the surgical adhesive and were inserted into the internal auditory canal. Additional adhesive was used to obtain a liquid-tight seal to prevent leakage of the cerebro-spinal fluid since the auditory nerve was removed in this procedure.

The use of the present surgical adhesive facilitated quicker healing of TM and canal incisions. Further, it was noted that in an informal comparison of MIPs used with and without the surgical adhesive, a proportionately higher number of slipped prostheses occurred without the use of the adhesive to anchor them in place.

The only disadvantages noted were that there may be a sensitivity reaction of the patient to the collagen component. Current reaction rates to Zyderm I were approximately 2%.

The data demonstrated the following advantages of the present surgical adhesive formulation over various prior art autogeneous fibrinogen glue (AFG) formulations. The surgical adhesive had a higher viscosity than AFG, which allowed more precise placement of the glue and clean-up of excess material. The viscosity was varied depending on the amount of collagen added to provide for a variety of applications. The whitish color of the surgical adhesive stands out in the surgical field unlike AFG which is reddish or straw in color.

A greater adhesiveness and overall durability permitted rougher handling of the adhesive. In vitro data showed greater persistence of the clot allowing for better healing. The surgical adhesive was a good tissue filler in the plastic reconstructive aspects of ear surgery. A lower viscosity material (such as found in formulations without the collagen) could not be used to adequately hold a meatoplasty with packing.

The surgical adhesive was much easier and quicker to prepare than current AFG formulations, especially when plasma was used instead of cryoprecipitate as in Phase II of the Clinical Trials. In fact, the surgical adhesive was prepared in the OR as the patient was undergoing anesthesia, unlike current AFG formulations which require overnight processing. The surgical adhesive used far less of the patient's blood than current AFG formulations, which require at least about 40 to 80 cm³. In contrast, only 5 cm³ of plasma were required for quantities sufficient for middle ear surgery with bone and/or soft tissue reconstruction. A topical antimicrobial agent can be added to the surgical adhesive for percutaneous or external flap closure or augmentation.

All publications and patent applications mentioned in this specification are indicative of the level of skill of those skilled in the art to which this invention pertains.

The invention now being fully described, it will be apparent to one of ordinary skill in the art that many changes and modifications can be made thereto without departing from the spirit or scope of the appended claims.

## Claims

1. A surgical adhesive useful in treating a patient comprising in an aqueous composition:
(a) plasma from said patient;
(b) collagen at at least 5mg/ml and in an amount which is greater than the amount of fibrinogen in the composition and sufficient to thicken said composition; and
(c) thrombin to catalyze polymerization of fibrinogen present in said plasma to produce a clot.

2. A surgical adhesive useful in treating a patient comprising in an aqueous composition:
(a) plasma from said patient;
(b) collagen in a concentration of from 5mg/ml to 30mg/ml; and
(c) from 1 to 1000 NIHu thrombin.

3. The surgical adhesive according to claim 1 or claim 2 wherein said collagen concentration is from 10mg/ml to 20mg/ml.

4. The surgical adhesive according to claim 1, claim 2 or claim 3 additionally comprising an anti-fibrinolytic agent.

5. The surgical adhesive according to any one of the preceding claims additionally comprising an antimicrobial agent.

6. The surgical adhesive according to any one of the preceding claims additionally comprising an inorganic mineral.

7. The surgical adhesive according to claim 6 wherein said mineral is hydroxyapatite.

8. The surgical adhesive according to claim 6 wherein said inorganic mineral is provided by bone powder or bone chips.

9. The surgical adhesive according to any one of the preceding claims additionally comprising osteoblasts from said patient.

10. A method of making a surgical adhesive for treating a patient comprising:
(a) mixing plasma from said patient with collagen in an amount of at least 5mg/ml, said amount being sufficient to thicken said plasma to form a thickened composition and being greater than the amount of fibrinogen in the composition;
(b) combining said thickened composition with thrombin to catalyze polymerization of fibrinogen present in said plasma and optionally an effective amount of an antifibrinolytic agent, whereby said fibrinogen is polymerized to form said surgical adhesive.

11. The method according to claim 10 wherein thrombin is present in a concentration of from 100 to 500 NIHu/ml.

12. A kit comprising in a first container flowable, dispersable collagen and in a second container, thrombin, optionally admixed with an antifibrinolytic agent.

## Patentansprüche

1. Chirurgisches Klebstoffmaterial, das sich zur Behandlung eines Patienten eignet, umfassend in einer wässerigen Zusammensetzung:
(a) Plasma vom Patienten;
(b) Kollagen von zumindest 5 mg/ml und in einer Menge, die größer ist als die Menge an Fibrinogen in der Zusammensetzung und ausreicht, die genannte Zusammensetzung zu verdicken; und
(c) Thrombin zum Katalysieren der Polymerisation des im genannten Plasma vorhandenen Fibrinogens, um Gerinnung hervorzurufen.

2. Chrirugisches Klebstoffmaterial, das sich zur Behandlung eines Patienten eignet, umfassend in einer wässerigen Zusammensetzung:
(a) Plasma vom Patienten;
(b) Kollagen in einer Konzentration von 5 mg/ml bis 30 mg/ml; und
(c) von 1 bis 1000 NIH-Thrombineinheiten.

3. Chirurgisches Klebstoffmaterial nach Anspruch 1 oder Anspruch 2, worin die Kollagen-Konzentration 10 mg/ml bis 20 mg/ml beträgt.

4. Chirurgisches Klebstoffmaterial nach Anspruch 1, Anspruch 2 oder Anspruch 3, das zusätzlich ein antifibrinolytisches Agens umfaßt.

5. Chirurgisches Klebstoffmaterial nach einem der vorhergehenden Ansprüche, das zusätzlich ein antimikrobielles Agens umfaßt.

6. Chirurgisches Klebstoffmaterial nach einem der vorhergehenden Ansprüche, das zusätzlich ein anorganisches Mineral umfaßt.

7. Chirurgisches Klebstoffmaterial nach Anspruch 6, worin das Mineral Hydroxyapatit ist.

8. Chirurgisches Klebstoffmaterial nach Anspruch 6, worin das anorganische Mineral durch Knochenpulver oder Knochenspäne bereitgestellt ist.

9. Chirurgisches Klebstoffmaterial nach einem der vorhergehenden Ansprüche, das zusätzlich Osteoblasten vom Patienten umfaßt.

10. Verfahren zum Herstellen eines chirugischen Klebstoffmaterials zur Behandlung eines Patienten umfassend:
(a) das Vermischen von Plasma vom Patienten mit Kollagen in einer Menge von zumindest 5 mg/ml, wobei die Menge ausreicht, das Plasma zu verdicken, um eine verdickte Zusammensetzung zu bilden und größer ist als die Menge an Fibrinogen in der Zusammensetzung;
(b) das Kombinieren der verdickten Zusammensetzung mit Thrombin, um die Polymerisation von im Plasma vorhandenen Fibrinogen zu katalysieren, und wahlweise mit einer wirksamen Menge eines antifibrinolytischen Agens, wodurch das genannte Fibrinogen polymerisiert wird, um das chirurgische Klebstoffmaterial zu bilden.

11. Verfahren nach Anspruch 10, worin Thrombin in einer Konzentration von 100 bis 500 NIH-Einheiten/ml vorhanden ist.

12. Satz umfassend in einem ersten Behälter fließfähiges, dispergierbares Kollagen und in einem zweiten Behälter Thrombin, wahlweise vermischt mit einem antifibrinolytischen Agens.

## Revendications

1. Adhésif chirurgical utile dans le traitement d'un patient comprenant dans une composition aqueuse :
(a) du plasma provenant dudit patient;
(b) du collagène à au moins 5mg/ml et en une quantité qui est supérieure à la quantité de fibrinogène dans la composition et suffisante pour épaissir ladite composition; et
(c) de la trombine pour catalyser la polymérisation du fibrinogène présent dans ledit plasma pour produire un caillot.

2. Adhésif chirurgical utile dans le traitement d'un patient comprenant dans une composition aqueuse :
(a) du plasma provenant dudit patient;
(b) du collagène à une concentration de 5 mg/ml à 30 mg/ml; et
(c) de 1 à 1000 NIHu thrombine.

3. Adhésif chirurgical selon la revendication 1 ou 2, dans lequel ladite concentration du collagène est de 10 mg/ml à 20 mg/ml.

4. Adhésif chirurgical selon la revendication 1, 2 ou 3, comprenant additionnellement un agent antifibrinolytique.

5. Adhésif chirurgical selon l'une quelconque des revendications précédentes comprenant additionnellement un agent anti-microbien.

6. Adhésif chirurgical selon l'une quelconque des revendications précédentes comprenant additionnellement un minéral inorganique.

7. Adhésif chirurgical selon la revendication 6, dans lequel ledit minéral est hydroxyapatite.

8. Adhésif chirurgical selon la revendication 6, dans lequel ledit minéral inorganique est fourni par de la poudre d'os ou des écailles d'os.

9. Adhésif chirurgical selon l'une quelconque des revendications précédentes comprenant additionnellement des ostéoblastes provenant dudit patient.

10. Méthode de fabrication d'adhésif chirurgical pour traiter un patient comprenant :
(a) le mélange du plasma provenant dudit patient avec du collagène en une quantité d'au moins 5 mg/ml, ladite quantité étant suffisante pour épaissir ledit plasma pour former une composition épaissie et étant supérieure à la quantité de fibrinogène dans la composition;
(b) la combinaison de ladite composition épaissie avec de la thrombine pour catalyser la polymérisation du fibrinogène présent dans ledit plasma et optionnellement une quantité efficace d'un agent antifibrinolytique, ce par quoi ledit fibrinogène est polymérisé pour former ledit adhésif chirurgical.

11. Méthode selon la revendication 10, dans laquelle la thrombine est présente à une concentration de 100 à 500 NIHu/ml.

12. Assortiment comprenant dans un premier conteneur du collagène écoulable, dispersable et dans un second conteneur, de la thrombine, optionnellement mélangée avec un agent antifibrinolytique.
